# EUROPEAN PATENT APPLICATION

(11) **EP 2 033 643 A1**
(43) Date of publication of application: **11.03.2009**
(21) Application number: 07767540.3
(22) Date of filing: 26.06.2007
(51) Int. Cl.: A61K 31/4178, A61K 9/14, A61K 9/16, A61K 9/20, A61P 9/12, A61P 43/00

(54) **COMPRESSED PREPARATION**

(30) Priority: 27.06.2006 JP 2006176146
(71) Applicant: Daiichi Sankyo Company, Limited, Chuo-ku Tokyo 103-0023 (JP)
(72) Inventor: YADA, Shuichi, Hiratsuka-shi, Kanagawa 245-0014 (JP); HASEGAWA, Susumu, Hiratsuka,-shi, Kanagawa 245-0014 (JP); TANIMOTO, Mitsuhide, Hiratsuka-shi, Kanagawa 254-0014 (JP)
(74) Representative: Wallace, Sheila Jane
(86) International application number: PCT/JP2007/062734
(87) International publication number: WO 2008/001734

(57) **Abstract**

[Object]

The present invention provides an olmesartan medoxomil-containing drug product having an improved dissolution property.

[Means for Solution]

A production method for an olmesartan medoxomil-containing drug product **characterized in that** the method comprises a process of compressing a composition.

## Description

### [TECHNICAL FIELD]

The present invention relates to a method for producing an olmesartan medoxomil-containing drug product having an improved dissolution property.

### [BACKGROUND ART]

Olmesartan medoxomil, which is an angiotensin II receptor antagonist, is useful as an active ingredient of a drug for treating or preventing hypertension. However, the production of olmesartan medoxomil-containing drug products requires formulation techniques which enhance the dissolution property of olmesartan medoxomil.

When producing a drug product containing a sparingly-soluble active ingredient, a formulation technique which enhances the dissolution property of the active ingredient, such as the addition of a solubilizer, is generally used. Meanwhile, a process of compressing a composition was regarded as an unfavorable technique in the case of producing a drug product containing a sparingly-soluble active ingredient, since the process delays the disintegrating speed of the drug product and thus worsens the dissolution property.
[Patent Document 1] Japanese Patent No. 2082519
[Patent Document 2] U.S. Patent No. 5616599
[Non-patent Document 1] J. Med. Chem., 39, 323-338 (1996)
[Non-patent Document 2] Annu. Rep. Sankyo Res. Lab. (Sankyo Kenkyusho Nempo) 55, 1-91 (2003)

### [DISCLOSURE OF THE INVENTION]

### [PROBLEM TO BE SOLVED BY THE INVENTION]

An object of the present invention is to provide an olmesartan medoxomil-containing drug product having an improved dissolution property.

### [MEANS FOR SOLVING THE PROBLEM]

As a result of conducting extensive studies on formulation techniques which would improve the dissolution property of olmesartan medoxomil, the inventors of the present invention surprisingly found that adoption of a process of compressing a composition, which was considered to worsen the dissolution property, instead improved the dissolution property, thereby leading to completion of the present invention.

The present invention provides a method for producing an olmesartan medoxomil-containing drug product characterized in that the method comprises a process of compressing a composition.

The present invention includes the following.
(1) A method for producing an olmesartan medoxomil-containing drug product characterized in that the method comprises a process of compressing a composition.
(2) The production method according to (1), wherein the process of compressing the composition applies a pressure of 20 N/mm² or higher.
(3) The production method according to (1), wherein the process of compressing the composition applies a pressure of 40 to 600 N/mm².
(4) The production method according to (1), wherein the process of compressing the composition applies a pressure of 60 to 400 N/mm².
(5) The production method according to (1) to (4), wherein the process of compressing the composition is a tableting process.
(6) The production method according to (1) to (4), wherein the drug product is powders, fine particles or granules.
(7) The production method according to (1) to (5), wherein the drug product is tablets.
(8) The production method according to (1) to (7), wherein the composition comprises a dissolution improving agent.
(9) An olmesartan medoxomil-containing drug product produced by the method according to (1) to (8).

### [EFFECT OF THE INVENTION]

According to the present invention, a method can be provided for producing an olmesartan medoxomil-containing drug product characterized in that the method comprises a process of compressing a composition.

### [BEST MODE FOR CARRYING OUT THE INVENTION]

Olmesartan medoxomil, which is an active ingredient used in the production method of the present invention, can be easily produced in accordance with the methods described in Japanese Patent No. 2082519 (U.S. Patent No. 5,616,599).

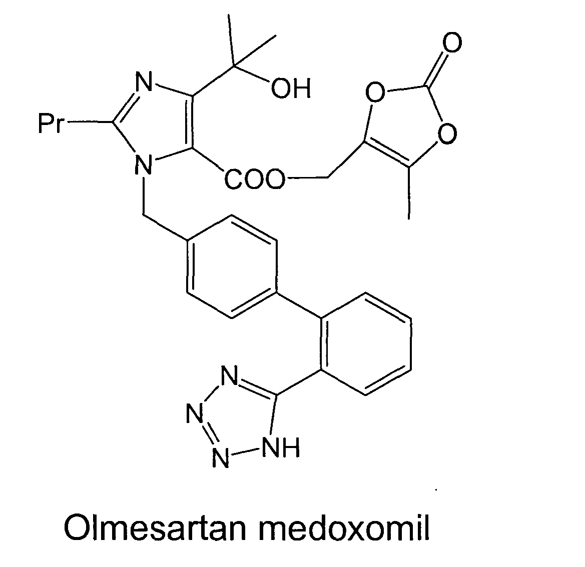

So long as it comprises the "process of compressing a composition", there is no particular limitation on other processes with respect to the method for producing an olmesartan medoxomil-containing drug product according to the present invention, and production can be carried out by using general methods described in publications such as Powder Technology and Pharmaceutical Processes (D. Chulia et al, Elsevier Science Pub Co (December 1, 1993)).

The "process of compressing a composition" of the present invention has no limitation with respect to the means which applies pressure, so long as the process can apply externally a mechanical pressure to a composition containing olmesartan medoxomil. For example, a process which mixes or stirs a composition by applying mechanical pressure, a compressing process which is carried out to granulate a composition mixture, a pulverizing process to pulverize the composition by mechanical pressure or by shear force, a tableting process to compress and mold the composition into a tablet and the like can be mentioned. Preferably, the "process of compressing a composition" is a tableting process to make a tablet.

Although the amount of pressure applied to the composition in the "process of compressing a composition" is not particularly limited so long as it is an amount capable of enhancing the dissolution property of the active ingredient, it is preferably a pressure equivalent to 20 N/mm² or higher, more preferably a pressure equivalent to 40 to 600 N/mm², and most preferably a pressure equivalent to 60 to 400 N/mm².

The drug product of the present invention can, if necessary, contain additives such as suitable pharmacologically acceptable excipients, lubricants, binders, disintegrants, emulsifiers, stabilizers, flavoring agents or diluents.

As for the "excipients" used, organic excipients including sugar derivatives such as lactose, sucrose, glucose, mannitol or sorbitol; starch derivatives such as corn starch, potato starch, α-starch or dextrin; cellulose derivatives such as crystalline cellulose; gum Arabic; dextran; and pullulan; and inorganic excipients including silicate derivatives such as light anhydrous silicic acid, synthetic aluminum silicate, calcium silicate or magnesium metasilicate aluminate; phosphates such as dibasic calcium hydrogenphosphate; carbonates such as calcium carbonate; and sulfates such as calcium sulfate can be mentioned.

As for the "lubricants" used, stearic acid; stearic acid metal salts such as calcium stearate or magnesium stearate; talc; colloidal silica; waxes such as beeswax or spermaceti; boric acid; adipic acid; sulfates such as sodium sulfate; glycol; fumaric acid; sodium benzoate; D,L-leucine; lauryl sulfates such as sodium lauryl sulfate or magnesium lauryl sulfate; silicic acids such as silicic anhydride or silicate hydrate; and the aforementioned starch derivatives can be mentioned.

As for the "binders" used, hydroxypropyl cellulose, hydroxypropyl methyl cellulose, polyvinylpyrrolidone, macrogol and compounds similar to the aforementioned excipients can be mentioned.

As for the "disintegrants" used, cellulose derivatives such as low-substituted hydroxypropyl cellulose, carboxymethyl cellulose, calcium carboxymethyl cellulose or internally crosslinked sodium carboxymethyl cellulose; cross-linked polyvinylpyrrolidone; or chemically modified starches/celluloses such as carboxymethyl starch or sodium carboxymethyl starch can be mentioned.

As for the "emulsifiers" used, colloidal clays such as bentonite or bee gum; metal hydroxides such as magnesium hydroxide or aluminum hydroxide; anionic surfactants such as sodium lauryl sulfate or calcium stearate; cationic surfactants such as benzalkonium chloride; or nonionic surfactants such as polyoxyethylene alkyl ether, polyoxyethylene sorbitan fatty acid ester or sucrose fatty acid ester can be mentioned.

As for the "stabilizers" used, para-hydroxybenzoic acid esters such as methyl paraben or propyl paraben; alcohols such as chlorobutanol, benzyl alcohol or phenyl ethyl alcohol; benzalkonium chloride; phenols such as phenol or cresol; thimerosal; dehydroacetic acid; or sorbic acid can be mentioned.

As for the "flavoring agents" used, sweeteners such as sodium saccharin or aspartame; sour flavourings such as citric acid, malic acid or tartaric acid; or fragrances such as menthol, lemon or orange fragrance can be mentioned.

As for the "diluents" used, lactose, mannitol, glucose, sucrose, calcium sulfate, calcium phosphate, hydroxypropyl cellulose, microcrystalline cellulose, water, ethanol, polyethylene glycol, propylene glycol, glycerol, starch, polyvinylpyrrolidone, magnesium metasilicate aluminate, or mixtures thereof can be mentioned.

The present invention is characterized in that the dissolution property of the active ingredient can be improved without using a "solubilizer", by the adoption of the "process of compressing a composition". However, the drug product of the present invention may include a "solubilizer", thereby enabling the production of a drug product having a further improved dissolution property.

As for the "solubilizer" used, water-soluble polymers, surfactants and the like can be mentioned.

As for the "water-soluble polymers", there can be mentioned for example, cellulose derivatives such as hydroxypropyl methyl cellulose, methyl cellulose, hydroxypropyl cellulose and sodium carboxymethyl cellulose; synthetic polymers such as polyvinylpyrrolidone, aminoalkyl methacrylate copolymer, carboxyvinyl polymer, polyvinyl alcohol and macrogol; HA "Sankyo", gum Arabic, agar, gelatin and sodium alginate, preferably hydroxypropyl methyl cellulose, methyl cellulose, hydroxypropyl cellulose, sodium carboxymethyl cellulose, HA "Sankyo", polyvinylpyrrolidone, and polyvinyl alcohol, more preferably hydroxypropyl methyl cellulose, methyl cellulose, hydroxypropyl cellulose, and sodium carboxymethyl cellulose, and particularly preferably methyl cellulose and/or hydroxypropyl cellulose. In the present invention, these can be used alone, or can be used by combining two or more types. The water-soluble polymer may be included preferably in the range of 1 to 90 wt % of the formulation weight, and more preferably in the range of 5 to 85 wt %.

As for the "surfactants", sodium lauryl sulfate, polyethylene glycol, polysorbate 80 and the like can be mentioned. In the present invention, these can be used alone, or can be used by combining two or more types.

Further, in the present invention, other active ingredients may be included if necessary. As for such active ingredients, there can be mentioned for example, diuretic drugs such as Hydrochlorothiazide, Methylclothiazide, Benzylhydrochlorothiazide, Trichloromethiazide, Cyclopenthiazide, Polythiazide, Ethiazide, Cyclothiazide, Bendroflumethiazide and Hydroflumethiazide; calcium antagonists such as Azelnidipine, Amlodipine, Benidipine, Nitrendipine, Manidipine, Nicardipine, Nifedipine, Nisoldipine, Cilnidipine, Lercanidipine, Nimodipine, Aranidipine, Efonidipine, Barnidipine, Felodipine and Nilvadipine; insulin resistance alleviating agents such as Pioglitazone, Rosiglitazone, Rivoglitazone, MCC-555, NN-2344, BMS-298585, AZ-242, LY-519818 and TAK-559; HMG-CoA reductase inhibitors such as Pravastatin, Simvastatin, Atorvastatin, Rosuvastatin, Cerivastatin, Pitavastatin and Fluvastatin; ACAT inhibitors such as SMP-797 and Pactimibe; and the like. Here, they are not limited to these.

The amount of these active ingredients is not particularly limited, and an amount generally used for tablets may be used.

As for the "drug product" of the present invention, tablets (including sublingual tablets and tablets that disintegrate in the mouth), capsules (including soft capsules and microcapsules) granules, fine particles, powders, pills and troches can be mentioned for example, and is preferably powders, fine particles, granules, capsules or tablets, and most preferably tablets.

The tablets of the present invention can be obtained by granulating a base drug with an excipient, binder and the like, drying and screening, mixing the resulting granules with a lubricant and the like, and then forming them into tablets, which is a method known per se. Here, granulation can be conducted by any one of wet granulation, dry granulation or heat granulation, and in particular, it is conducted by using a high speed mixing granulator, fluid bed dryer, extrusion granulator or roller compactor for example. In addition, after granulation, operations such as drying and regulating the granules may be conducted if necessary. A mixture of base drug, excipient, binder, lubricant and the like can also be directly formed into tablets.

Here, granulation refers to an operation which makes granules having a nearly uniform shape and size from raw materials which are in a form such as powders, lumps, solutions or molten liquids. Granulation includes procedures which provide finished products such as granules, powders and fine particles, and procedures in which intermediate products are produced for subsequent use in the manufacture of tablets, capsules and the like.

The granulated products thus obtained can be regulated to a desired particle size, and can be formed into a drug product in the form of powders, fine particles or granules. These drug products may also be made as capsules by filling them into capsules, or they may be further supplemented with a disintegrant, lubricant and the like if necessary and made into a drug product in the form of tablets by compression molding by a tableting machine. Operations of mixing and granulation are both widely used in the field of formulation techniques, and those skilled in the art can carry them out appropriately. In addition, at least one layer of a film coating may be provided over the tablets.

Coating is, for example, conducted by using a film coating machine. As film coating bases, sugar coating bases, water-soluble film coating bases, enteric film coating bases and sustained release film coating bases can be mentioned for example.

As for the "sugar coating bases", saccharose is used, and it can be used in combination with one or more additives selected from talc, precipitated calcium carbonate, calcium phosphate, calcium sulfate, gelatin, gum Arabic, polyvinylpyrrolidone and pullulan.

As for the "water-soluble film coating bases", cellulose derivatives such as hydroxypropyl cellulose, hydroxypropyl methyl cellulose, hydroxyethyl cellulose, methyl hydroxyethyl cellulose and sodium carboxymethyl cellulose; synthetic polymers such as polyvinyl acetal diethyl aminoacetate, aminoalkyl methacrylate copolymer and polyvinylpyrrolidone; and polysaccharides such as pullulan can be mentioned.

As for the "enteric film coating bases", cellulose derivatives such as hydroxypropyl methyl cellulose phthalate, hydroxypropyl methyl cellulose acetate succinate, carboxymethyl ethyl cellulose and cellulose acetate phthalate; acrylic acid derivatives such as methacrylic acid copolymer L, methacrylic acid copolymer LD and methacrylic acid copolymer S; and natural substances such as shellac can be mentioned.

As for the "sustained release film coating bases", cellulose derivatives such as ethyl cellulose; and acrylic acid derivatives such as aminoalkyl methacrylate copolymer RS, ethyl acrylate-methyl methacrylate copolymer emulsion can be mentioned.

The above coating base may be used by mixing two or more different coating bases in a suitable ratio. In addition, the coating bases may also contain suitable pharmacologically acceptable additives such as plasticizers, excipients, lubricants, opacifying agents, colorants or antiseptics, if necessary.

The dosage amount of olmesartan medoxomil, which is an active ingredient of the drug product according to the present invention, may vary depending on various factors such as the symptoms, age, and body weight of a patient. Although its dosage amount varies depending on the symptoms, age and the like, an adult human is generally administered orally with 5 to 40 mg, once a day. Preferably, a tablet containing 5 mg, 10 mg, 20 mg or 40 mg is administered orally once a day.

The drug product of the present invention is effective for the prevention or treatment of hypertension or diseases caused by hypertension (more specifically, hypertension, heart diseases [angina pectoris, myocardial infarction, arrhythmia, cardiac insufficiency or cardiac hypertrophy], kidney diseases [diabetic nephropathy, glomerular nephritis or nephrosclerosis], or cerebrovascular disease [cerebral infarction or cerebral hemorrhage] and the like.

### [EXAMPLES]

The present invention will be described in more detail by way of examples and the like; however, the present invention is not intended to be limited to these.

### (Example A-1) Formulation A, tableting pressure 28 N/mm² (punch: 9.5 mm diameter flat faced punch)

The components except for magnesium stearate listed in formulation A were mixed in a mortar, followed by addition of magnesium stearate, and the components were blended in a bag, thereby obtaining a powder mixture for tableting. The obtained powder mixture for tableting was tableted with a tableting pressure of 28 N/mm².

| Formulation A | |
|---|---|
| Olmesartan medoxomil | 20 mg |
| Lactose | 106 mg |
| L-HPC | 20 mg |
| HPC-L | 3 mg |
| Avicel | 10 mg |
| Magnesium stearate | 1 mg |
| | 160 mg |

### (Example A-2) Formulation A, tableting pressure 85 N/mm² (punch: 9.5 mm diameter flat faced punch)

The powder mixture for tableting obtained in Example A-1 was tableted with a tableting pressure of 85 N/mm².

### (Example A-3) Formulation A, tableting pressure 141 N/mm² (punch: 9.5 mm diameter flat faced punch)

The powder mixture for tableting obtained in Example A-1 was tableted with a tableting pressure of 141 N/mm².

### (Example A-4) Formulation A, tableting pressure 141 N/mm² (punch: 9.5 mm diameter flat faced punch), pulverization

The tablet obtained in Example A-3 was pulverized in a mortar.

### (Example B-1) Formulation B, tableting pressure 28 N/mm² (punch: 9.5 mm diameter flat faced punch)

The components except for magnesium stearate listed in formulation B were mixed in a mortar, followed by addition of magnesium stearate and the components were blended in a bag, thereby obtaining a powder mixture for tableting. The obtained powder mixture for tableting was tableted with a tableting pressure of 28 N/mm².

| Formulation B | |
|---|---|
| Olmesartan medoxomil | 20 mg |
| Erythritol | 139.5 mg |
| D-mannitol | 25 mg |
| Aspartame | 7.5 mg |
| HPC-SSL | 6 mg |
| Magnesium stearate | 2 mg |
| | 200 mg |

### (Example B-2) Formulation B, tableting pressure 85 N/mm² (punch: 9.5 mm diameter flat faced punch)

The powder mixture for tableting obtained in Example B-1 was tableted with a tableting pressure of 85 N/mm².

### (Example B-3) Formulation B, tableting pressure 141 N/mm² (punch: 9.5 mm diameter flat faced punch)

The powder mixture for tableting obtained in Example B-1 was tableted with a tableting pressure of 141 N/mm².

### (Example B-4) Formulation B, tableting pressure 141 N/mm² (punch: 9.5 mm diameter flat faced punch), pulverization

The tablet obtained in Example B-3 was pulverized in a mortar.

### (Comparative Example C-1) Formulation A, mixing

The powder mixture for tableting obtained in Example A-1 was used.

### (Comparative Example C-2) Formulation B, mixing

The powder mixture for tableting obtained in Example B-1 was used.

### (Comparative Example C-3) Bulk drug of olmesartan medoxomil

A bulk drug of olmesartan medoxomil was used.

Dissolution properties were measured for the drug products as obtained in the Examples and Comparative Examples by the following method, and the results are shown in the Table.

### (Test Example)

Testing was carried out in accordance with Method 2 of the Dissolution Test (Paddle Method) described in the 14th Revised Edition of the Japanese Pharmacopoeia, at the paddle revolution speed of 50 revolutions per minute and using 900 mL of Japanese Pharmacopoeia Solution 2 (JP-2) for the test solution. The test solution was sampled at 30 minutes and 60 minutes after the start of testing, and the dissolution rate of olmesartan medoxomil was measured by absorptiometry. [Toyama Sangyo Co., Lid. : DISSOLUTION TESTER NTR-6000, Shimadzu Corporation : SPECTROPHOTOMETER UV-1600] Tests were carried out for two tablets, and average values are shown.

**(Table 1)**

| | Dissolution rate (%) | |
|---|---|---|
| Specimen | After 30 minutes | After 60 minutes |
| Example A-1 | 65.7 | 77.5 |
| Example A-2 | 77.9 | 87.4 |
| Example A-3 | 83.1 | 90.3 |
| Example A-4 | 79.1 | 82.9 |
| Example B-1 | 61.8 | 74.3 |
| Example B-2 | 71.4 | 82.5 |
| Example B-3 | 81.2 | 91.4 |
| Example B-4 | 83.2 | 91.2 |
| Comparative Example C-1 | 60.6 | 71.0 |
| Comparative Example C-2 | 58.5 | 71.0 |
| Comparative Example C-3 | 60.0 | 68.4 |

As shown in Table 1, drug products to which the "process of compressing a composition" (tableting process) was applied provided superior dissolution properties when compared to drug products to which the "process of compressing a composition" (tableting process) was not applied or compared to the bulk drug.

### [INDUSTRIAL APPLICABILITY]

According to the present invention, an olmesartan medoxomil-containing drug product having an improved dissolution property, can be obtained.

## Claims

1. A method for producing an olmesartan medoxomil-containing drug product **characterized in that** the method comprises a process of compressing a composition.

2. The production method according to claim 1, wherein the process of compressing the composition applies a pressure of 20 N/mm² or higher.

3. The production method according to claim 1, wherein the process of compressing the composition applies a pressure of 40 to 600 N/mm².

4. The production method according to claim 1, wherein the process of compressing the composition applies a pressure of 60 to 400 N/mm².

5. The production method according to claims 1 to 4, wherein the process of compressing the composition is a tableting process.

6. The production method according to claims 1 to 4, wherein the drug product is powders, fine particles or granules.

7. The production method according to claims 1 to 5, wherein the drug product is tablets.

8. The production method according to claims 1 to 7, wherein the composition comprises a dissolution improving agent.

9. An olmesartan medoxomil-containing drug product produced by the method according to claims 1 to 8.
